# EUROPEAN PATENT APPLICATION

(11) **EP 4 000 644 A1**
(43) Date of publication of application: **25.05.2022**
(21) Application number: 21208820.7
(22) Date of filing: 17.11.2021
(51) Int. Cl.: A61L 2/08, A61L 2/10, H05B 47/00

(54) **INTEGRATED UV DISINFECTION**

(30) Priority: 19.11.2020 US 202016952320
(71) Applicant: Palo Alto Research Center Incorporated, Webster, NY 14580 (US)
(72) Inventor: WUNDERER, Thomas, Santa Cruz, 95062 (US); MARTINI, Joerg, San Francisco, 94107 (US); MAEDA, Patrick Y, Mountain View, 94040 (US); CROMARTY, Barbara, Boulder Creek, 95006 (US); FAUTLEY, Paloma, San Jose, 95128 (US)
(74) Representative: Gill Jennings & Every LLP

(57) **Abstract**

Methods and systems for disinfecting a surface, can include a light source, and a transparent window located above the light source. The light source can be integrated into an object, and an outer surface of the object can be located above the transparent window. Light from the light source can irradiate the outer surface through the transparent window and from within the object to disinfect the outer surface of the object. The light can comprise violet and ultraviolet (UV) light. A photocatalytic layer comprising a photocatalytic material may also be located above the transparent window and below the outer surface.

## Description

### TECHNICAL FIELD

Embodiments are generally related to systems and methods for the disinfection and decontamination of surfaces. Embodiments also relate to the use of ultraviolet (UV) light for surface disinfection and decontamination.

### BACKGROUND

Triggered by the recent developments related to COVID-19, people are particularly sensitive to shared spaces and objects that can potentially act as a medium for transmitting pathogens. Automatically disinfecting 'high-touch' surfaces is a greatly desired function that does not currently exist. High-touch surfaces can include, for example, various areas and surfaces in public (and private) that may be frequently touched by people. Examples of such surfaces include handlebars, elevator buttons, traffic lights, shared touch screens (e.g., associated with a coffee machine, a cashier check-out, an ATM machine, etc), light switches, and so on. High-touch surfaces may also include surfaces in areas that may not be frequently touched, but which may require frequent disinfection in general (e.g., in a surgery room).

A conventional approach for sanitizing surfaces can involve exposing a surface to UV light, which is harmful to potential microorganisms, such as bacteria, viruses, and fungi, which may be present on the surface. The UV light should be at sufficiently short wavelengths to breakdown and eradicate these organisms, thereby disinfecting the surface where these organisms may be present. The short wavelength radiation can destroy such organisms at a micro-organic level. The UV light can destroy nucleic acids, for example, in these organisms' DNA. Once the DNA (or RNA) chain is disrupted, the organisms are unable to replicate, thereby eliminating the harm from these pathogens.

The inventors have developed a solution to the problem of disinfecting high-touch surfaces through the implementation of self-sanitization measures that include the use of UV photons or photon-enabled chemical processes as discussed in greater detail herein.

### BRIEF SUMMARY

The following summary is provided to facilitate an understanding of some of the innovative features unique to the disclosed embodiments and is not intended to be a full description. A full appreciation of the various aspects of the embodiments disclosed herein can be gained by taking the entire specification, claims, drawings, and abstract as a whole.

It is, therefore, one aspect of the disclosed embodiments to provide a method and system for disinfecting a surface.

It is a further aspect of the disclosed embodiments to provide a method and system for automatically disinfecting high-touch surfaces.

It is another aspect of the disclosed embodiments to provide a method and system for the 'on demand' disinfection of a surface of an object by illuminating the surface with light from within the object.

It is yet another aspect of the disclosed embodiments to provide a method and system for disinfecting a surface through self-sanitization measures using UV photons or photon-enabled chemical processes.

The aforementioned aspects and other objectives and advantages can now be achieved as described herein. In an embodiment, a system for disinfecting a surface can include a light source, and a transparent window located above the light source. The light source can be integrated into an object, and an outer surface of the object can be located above the transparent window. Light from the light source can irradiate the outer surface through the transparent window and from within the object to disinfect the outer surface of the object. The light can comprise violet light or ultraviolet (UV) light.

In an embodiment of the system, a photocatalytic layer can comprise a photocatalytic material, and can be located above the transparent window and below the outer surface.

In an embodiment of the system, the photocatalytic layer can comprise at least one of, for example, ZnO, TiO₂, Fe₂O₃, WO₃, In₂O₃, Ag, or a nonmetallic semiconductor material.

In an embodiment of the system, the UV light can comprise at least one of, for example, UV-A light photons or UV-C light photons (i.e., having an emission in a range of between 100nm to 280nm).

In an embodiment of the system, the light source can comprise one or more of, for example, a semiconductor-based optoelectronic device or a gas discharge lamp.

In an embodiment of the system, the UV light can disinfect the outer surface by one or more of: inactivating germs on the outer surface of the object through reactive species created on the outer surface or damaging of nucleic acids associated with the germs, wherein the germs comprise a least one of: bacteria, virus, or a fungi; inducing antimicrobial properties in the outer surface of the object; or inducing microbicidal properties in the outer surface of the object.

In an embodiment of the system, the light source can comprise a plurality of UV light emitting diodes, and each UV light emitting diode among the plurality of UV light emitting diodes can comprise a III-Nitride based UV light emitting diode.

An embodiment of the system can further include an isolator layer, and an electrode layer comprising a semi-transparent material, wherein the transparent window is located above the electrode layer, and the light source is disposed in the isolator layer and at least partially within the electrode layer.

In an embodiment of the system, the photocatalytic material of the photocatalytic layer can comprise a photocatalytic coating, wherein the microbicidal properties can be induced in the outer surface when the UV light is absorbed in the photocatalytic material.

An embodiment of the system can further include an upconversion layer that can facilitate the use of visible light to generate UV photons in the upconversion layer.

An embodiment of the system can further include a fluorescence indication layer or a luminescence indication layer that can provide a visible indicator of UV photons emanating through the transparent window.

In an embodiment of the system, the light source can be automatically turned off when at least one person is detected within a predetermined distance of the light source.

In an embodiment of the system, an indication light can indicate when the outer surface of the objected has been disinfected.

An embodiment of the system can further include a substrate upon which a plurality of layers can be located including at least the light source, the transparent window, and the outer surface.

In an embodiment of the system, the substrate can comprise a flexible substrate.

In an embodiment of the system, the light source can further comprise at least one of the following: a back-illumination light source with respect to the object; or a side-coupling light source with respect to the object, wherein the back-illumination light source and the side-coupled light source each comprise at least one of: a structured optical surface or an optically structured film, wherein the structured optical surface or the optically structured film promote homogenization and uniform illumination across the photocatalytic surface.

In another embodiment, a system for disinfecting a surface, can include a substrate upon which a plurality of layers are located; a transparent window located above a light source, wherein the light source is integrated into an object; an outer surface of the object located above the transparent window; and a photocatalytic layer comprising a photocatalytic material, the photocatalytic layer located above the transparent window and below the outer surface, wherein the plurality of layers includes at least the light source, the transparent window, the photocatalytic layer, and the outer surface, and wherein light from the light source excites the outer surface through the transparent window and from within the object to disinfect the outer surface of the object, the light comprising violet light or ultraviolet (UV) light.

In an embodiment, a method for disinfecting a surface, can involve: exciting an outer surface of an object with light from a light source through a transparent window and from within the object, wherein the transparent window is located above the light source and the light source is integrated into the object, the outer surface of the object located above the transparent window, the light comprising violet light or ultraviolet (UV) light.

### BRIEF DESCRIPTION OF THE DRAWINGS

The accompanying figures, in which like reference numerals refer to identical or functionally-similar elements throughout the separate views and which are incorporated in and form a part of the specification, further illustrate the present invention and, together with the detailed description of the invention, serve to explain the principles of the present invention.
FIG. 1A and FIG. 1B illustrate graphs depicting experimental data indicative of the photocatalytic inactivation of influenza virus (IFV) and feline calicivirus (FCE), in accordance with an embodiment.
FIG. 2 illustrates a side view of a system for disinfecting a surface, in accordance with an embodiment;
FIG. 3 illustrates a side view of a system for disinfecting a surface, wherein the system includes side illumination of the surface, in accordance with an embodiment;
FIG. 4 illustrates a side view of a system for disinfecting a surface, wherein the system includes side illumination of the surface and an indicator light for status display, in accordance with an embodiment;
FIG. 5 illustrates a side view of a system for disinfecting a surface, wherein the system includes LED illumination of the surface, in accordance with an embodiment;
FIG. 6 illustrates a side view of a system for disinfecting a surface, wherein the system includes LED illumination with a fluorescent indicator for status display, in accordance with an embodiment; and
FIG. 7 illustrates a pictorial representation of a system for disinfecting a surface, in accordance with an embodiment.

### DETAILED DESCRIPTION

The particular values and configurations discussed in these non-limiting examples can be varied and are cited merely to illustrate one or more embodiments and are not intended to limit the scope thereof.

Subject matter will now be described more fully hereinafter with reference to the accompanying drawings, which form a part hereof, and which show, by way of illustration, specific example embodiments. Subject matter may, however, be embodied in a variety of different forms and, therefore, covered or claimed subject matter is intended to be construed as not being limited to any example embodiments set forth herein; example embodiments are provided merely to be illustrative. Likewise, a reasonably broad scope for claimed or covered subject matter is intended. Among other things, for example, subject matter may be embodied as methods, devices, components, or systems. Accordingly, embodiments may, for example, take the form of hardware, software, firmware, or any combination thereof (other than software per se). The following detailed description is, therefore, not intended to be interpreted in a limiting sense.

Throughout the specification and claims, terms may have nuanced meanings suggested or implied in context beyond an explicitly stated meaning. Likewise, phrases such as "in one embodiment", "in an embodiment", "in an example embodiment", or "in some embodiments" and variations thereof as utilized herein may or may not necessarily refer to the same embodiment. The phrase "in another embodiment", "in another example embodiment", or "in an alternative embodiment" and variations thereof as utilized herein may or may not necessarily refer to a different embodiment. It is intended, for example, that claimed subject matter may include combinations of embodiments in whole or in part.

In general, terminology may be understood, at least in part, from usage in context. For example, terms such as "and," "or," or "and/or" as used herein may include a variety of meanings that may depend, at least in part, upon the context in which such terms are used. Typically, "or" if used to associate a list, such as A, B, or C, is intended to mean A, B, and C, here used in the inclusive sense, as well as A, B, or C, here used in the exclusive sense. In addition, the terms "one or more" or "at least one" as used herein, depending at least in part upon context, may be used to describe any feature, structure, or characteristic in a singular sense or may be used to describe combinations of features, structures, or characteristics in a plural sense. Similarly, terms such as "a," "an," or "the", again, may be understood to convey a singular usage or to convey a plural usage, depending at least in part upon context. In addition, the term "based on" may be understood as not necessarily intended to convey an exclusive set of factors and may, instead, allow for existence of additional factors not necessarily expressly described, again, depending at least in part on context.

The disclosed embodiments address the need for disinfecting surfaces of objects through the implementation of self-sanitization measures using UV photons or photon-enabled chemical processes. As will be discussed in more detail herein, embodiments can be implemented, which can incorporate the use of photocatalytic material such as TiO₂ coatings, for example.

TiO₂ coatings have been used in a variety of applications including tiles in, for example, surgery rooms and surfaces on buses and trains to maintain cleanliness in these spaces. While TiO₂ is durable and easily cleanable and even exhibits some photocatalytic cleaning properties under visible (omnipresent) light, TiO₂ may only develop its full cleaning potential when illuminated with UV-A light. This may involve an external treatment with UV-A light that can require a great deal of time and manual labor. With the disclosed approach, however, external treatment with UV-A light becomes obsolete because the UV-A illumination is integrated into the surface design and can be optimized, controlled and automated.

Some embodiments may use integrated III-Nitride based light emitting diodes (LED) as a light source, which can allow through their compact form factor, an easy integration into commonly used objects and interaction with surfaces. Some embodiments may use UV-C light sources emitting at wavelengths near 265nm or light emitters with emission in the near-UV at wavelength of -400nm or below in combination with photocatalytic coating layers. Other UV generating light sources, such as Xenon lamps, mercury arc lamps, may be used with the embodiments.

The use of UV light for disinfection purposes is a well-studied field and various products can be found in the market place today, which incorporate UV light for disinfection. Most of these products use photon emission in the UV-C spectral range. Various products may also use xenon or mercury lamps with part of their emission spectrum in the desired wavelength range and typically come with relatively large form factors (e.g., UV disinfection robots, air conditioners, water purifiers, etc.). More recently, LED-based products have entered the marketplace with recent improvements in LED device performance (e.g., portable and stationary water purification, handheld UV disinfection flash lamp, etc.).

The embodiments, however, are different and novel from these products in that the light source discussed herein can be based on a light source configured from small form factor LEDs that can be fully integrated into the actual objects exposing the surfaces that may require decontamination. For example, a high-touch object such as a light switch can include an integrated UV LED as back-illumination light source for automatic disinfection of the front surface. In the case of UV-A LEDs, an additional photo-catalytic coating on the target surface will be added.

Additionally, the UV photon dosage requirements can be tailored to the specific need of the application (i.e., optical power and exposure time) and may be higher for de-contaminating viruses, for example, in comparison to bacteria. The required dosage might vary with the type of pathogen. We can consider the case for COVID-19 as an example implementation with a target exposure dose of UV-C light on the order of, for example, ∼15mJ/cm².

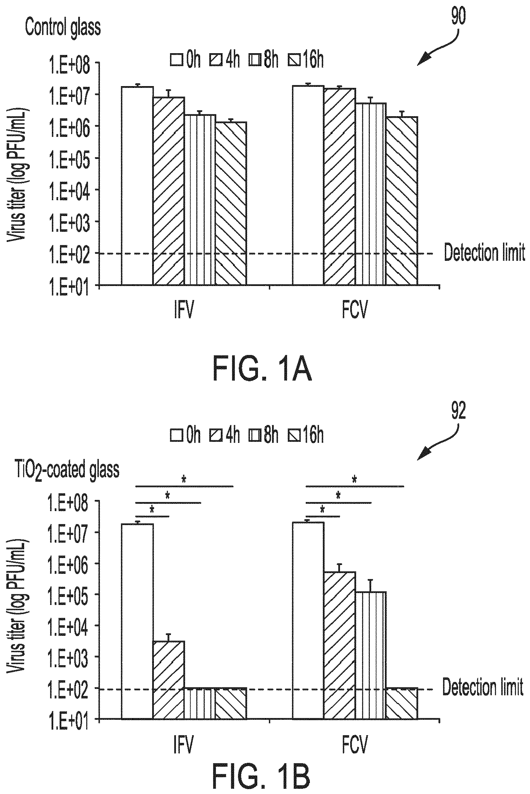

FIG. 1A and FIG. 1B illustrate a graph 90 and a graph 92 that depict experimental data indicative of the photocatalytic inactivation of influenza virus (IFV) and feline calicivirus (FCE). Note that Influenza Virus (IVF), an enveloped virus, has a phospholipid bilayer membrane with glycoprotein spikes surrounding the capsid. Feline calicivirus (FCV), a non-enveloped virus, has a capsid that consists of a protein and lacks a surrounding envelope.

Graph 90 displays data obtained through the use of a control glass and graph 92 shows the use of TiO₂-coated glass under backlight illumination (e.g., UV-A light intensity of 0.1 mW/cm²). The data depicted in graphs 90 and 92 represent the average values of three experiments (e.g., see Broad Spectrum Microbicidal Activity of Photocatalysis by TiO₂, Nakano et al., Catalysts 2013, 3, 310-323, which is incorporated herein by reference in its entirety). Graph 90 and graph 92 indicate that both bacterial and virological specimens can be inactivated on a photocatalytic surface as well as a general degradation of molecular species in contact with the film.

Note that the term 'photocatalytic' as utilized herein relates to the field of photocatalysis including semiconductor photocatalysis. Examples of photocatalysis applications and related research and materials are presented and discussed in the following non-limiting references, which are incorporated herein by reference in their entireties:
Andrew Mills and Stephen LeHunte, "An overview of semiconductor photocatalysis", Journal of Photochemistry and Photobiology A: Chemistry 108 (1997) 1-35
Kazuhito Hashimoto et al., "TiO2 Photocatalysis: A Historical Overview and Future Prospects", 2005 Jpn. J. Appl. Phys. 44 8269
Howard A. Foster, Iram B. Ditta, Sajnu Varghese, and Alex Steele, "Photocatalytic disinfection using titanium dioxide: spectrum and mechanism of antimicrobial activity", Appl Microbiol Biotechnol. 2011; 90(6): 1847-1868.

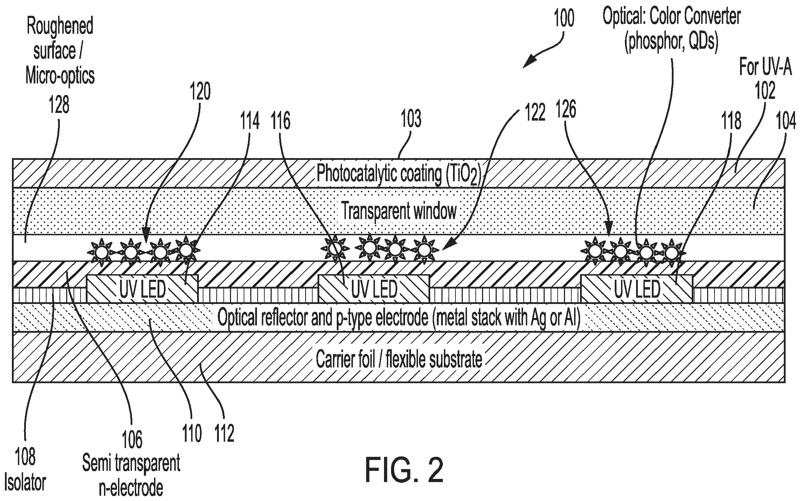

FIG. 2 illustrates a side view of a system 100 for disinfecting a surface 103, in accordance with an embodiment. The system 100 shown in FIG. 2 can include a light source comprising one or more light sources, such as, for example, a UV LED 114, a UV LED 115, and a UV LED 118. A transparent window 104 can be located above the light source (i.e., UV LED 114, a UV LED 115, and a UV LED 118), and the outer surface 103 can be located above the transparent window 104. Light from the light source can irradiate the outer surface 103 through the transparent window 104 to disinfect the outer surface 103. The system 100 can be integrated into an object having the outer surface 103.

The UV light can disinfect the outer surface 103 by, for example, inactivating germs on the outer surface 103 through reactive species created on the outer surface or damaging of nucleic acids associated with the germs (e.g., bacteria, virus, fungi, and other pathogens, etc.), inducing antimicrobial properties in the outer surface 103, or inducing microbicidal properties in the outer surface 103. Note that coatings that can contain an antimicrobial can inhibit the growth of these microorganisms, such as mildew and mold, protecting the film itself from degradation. On the other hand, microbicidal substances or compounds can go a step further by actually killing microscopic organisms on the surface 103.

Note that in some embodiments the light from the light source may include violet and ultraviolet (UV) light. That is, 405 nm may be the most attractive light source. This wavelength, however, does not necessarily provide UV light, which is typically considered less than 400nm. A preferred type of light that can be utilized with one or more embodiments can be referred to as *violet light and*/*or ultraviolet light.* Note that the term 'UV' light or 'ultraviolet' light as utilized herein may include both violet light and ultraviolet light in some embodiments, while in other embodiments the term 'UV' light or 'ultraviolet' light may only include UV light.

As utilized herein, the wavelength ranges of light can be named in the following manner: violet light can be electromagnetic radiation in a wavelength range of ∼400nm to ∼430nm. Ultraviolet light or UV light can be electromagnetic radiation in a wavelength range of 10nm to 400nm. More specifically, UV light in the wavelength range of 100nm to 280nm can be referred to as UV-C light. UV light in the wavelength range of 280nm to 315nm can be referred to as UV-B light. UV light in the wavelength range of 315nm to 400nm can be referred to as UV-A light.

In the embodiment shown in FIG. 2, the outer surface 103 can be a surface of a photocatalytic layer 102, which can be located above the transparent window 104 and below the outer surface 103. The photocatalytic layer 102 may be configured from a photocatalytic material such as, for example, ZnO, TiO₂, Fe₂O₃, WO₃, In₂O₃, Ag, or a nonmetallic semiconductor material. The photocatalytic material of the photocatalytic layer 102 can be implemented as a photocatalytic coating, wherein microbicidal properties are induced in the outer surface 103 when the UV light is absorbed in the photocatalytic material.

Note that photocatalytic surfaces can create mobile electron hole pairs upon absorption of photons with sufficient energy. The electrons or holes can then interact with the environment and form ions in the surrounding environment. These ions can either be considered reactive species or they can result in the creation of reactive species by downstream reactions. The term *reactive species* as utilized herein can refer to molecules that can have an adverse effect on any biomolecule, organism, microorganism, pathogen, virus, fungus, or germ exposed to the reactive species. Examples of 'reactive species' include O₂⁻ and OH.

The embodiment of system 100 depicted in FIG. 2 can be implemented as a UV-light disinfection sheet/blanket/tape. Multiple mini/-micro LEDs such as the UV LED 114, the UV LED 116, and the UV LED 118 can be implemented as a light source assembled on a flexible substrate 112, even in a digital manner if desired to accommodate specific form factors and for 'self-disinfection' of the exposed surface 103. Note that as utilized herein the term 'substrate' can relate to the base material on which processing can be conducted to produce new film or layers of material such as deposited coatings.

The system 100 can provide a disinfection function that can also be combined with other features of the object in which the system 100 may be incorporated. For example, touch screen displays may have integrated UV LEDs in combination with color converters for white light, such as a color converter 120, a color converter 122 and a color converter 126, which can provide the display and disinfection functionality, respectively. Furthermore, some white LEDs have a short wavelength (e.g., <400nm) excitation source in combination with wavelength converter phosphors. Such LEDs could be suitable for disinfection. In some embodiments visible indicators for process steps such as "disinfection in progress" and "disinfection is completed" can be provided to assure users of the cleanliness of the surface 103. Furthermore, an upconversion layer may be incorporated into system 100 so that visible light can be used to generate UV-A photons in the upconversion layer.

The system 100 can further include a combined reflector and electrode layer 106 that comprises an optical reflector and p-type electrode (e.g., a metal stack with Ag or Al). Furthermore, an isolator layer 108 may be disposed above the substrate 112 and below a semi-transparent n-electrode layer 106. The light source (e.g., UV LED 114, UV LED 116, and UV LED 118) can be partially disposed in the isolator layer 108 and the semi-transparent n-electrode layer 106.

In some embodiments, the UV LED 114, UV LED 116, and UV LED 118 may be implemented as integrated III-Nitride based light emitting diodes (LED) which allow through their compact form factor their integration into commonly used objects and interaction with surfaces. The light source can be implemented with either UV-C light sources emitting at wavelengths near 265nm or light emitters with emission in the near-UV at wavelength of ∼400nm or below in combination with photo-catalytic coating layers. Alternatively, in some embodiments, other UV generating light sources (e.g., Xenon lamps, mercury arc lamps, etc) can be used as the light source.

It can be appreciated that a number of different types light sources can be utilized as a light source, in accordance with varying embodiments. In some embodiments, group III-Nitride LEDs can be implemented as a preferred light source, as discussed above. In other embodiments, besides III-V semiconductors, a light source based on II-VI semiconductors may cover the UV/VIS spectral regime. This can include, for example, ZnS, ZnO, ZnSe and combinations thereof. However, these materials are typically not very robust, have issues related to doping, and coverage in the UV is quite limited. Thus, a preferred embodiment would include the use of group III-Nitride LEDs as a light source.

Some light sources may comprise a semiconductor diode implementation. There are other possible forms of UV light sources that can be adapted for use as a light source in accordance with alternative embodiments. For example, a light source may be implemented based on a semiconductor material pumped by other means than in a p-n-junction diode (e.g., e-beam pumping, gas discharge lamps, etc.). Gas based lamps may be implemented as a light source, for example, and may be configured in different form factors and compact arrangements.

The system 100 can disinfect its outer surface 103 'on demand' by illuminating the surface 103 with appropriate light from the inside. No manual labor or chemical is required, and no waste is generated. The disinfection can be electrically powered (e.g., battery, solar, or hard-wired) from within the object.

The system 100 can be configured as a self-disinfecting film stack with integrated III-Nitride based UV light emitting diodes (or other appropriate light source) exposing the high-touch surface 103. The UV light may either directly inactivate the germs (e.g., bacteria, viruses, fungi, etc.) in the case of UV-C light (e.g., λ∼265nm) or induce microbicidal properties when combining UV-A light (e.g., λ<400nm) with photocatalytic coatings such as TiO₂ or ZnO.

In the case of UV-C sources, the high-energy light can be effective in interacting and consequently damaging the RNA/DNA of the exposed species. Their reproduction can be hindered and the health-related harm eventually defeated. UV-C light is not naturally present on earth as most of the high-energy radiation from the sun is absorbed in the higher atmosphere. UV-C light can be damaging to human skin and eyes. Thus, safety measures should be implemented to avoid secondary implications through radiation.

Using UV-A light sources the potential risk to humans can be significantly reduced. Although the effectiveness for disinfection is typically reduced at the longer wavelengths, this situation can be changed with the implementation of a photo-catalytic coating to significantly enhance the effectiveness in inactivating the undesired germs. Such coatings can include, for example, TiO₂ films or TiO₂ films including Ag, or ZnO films. The UV light can be absorbed in such films creating electron-hole pairs that interact with oxygen or water molecules creating hydroxyl radicals that eventually inactivate the germs on the surface. Both bacterial and virological specimens can be inactivated on the photocatalytic surface (e.g., surface 103) as well as a general degradation of molecular species in contact with the film.

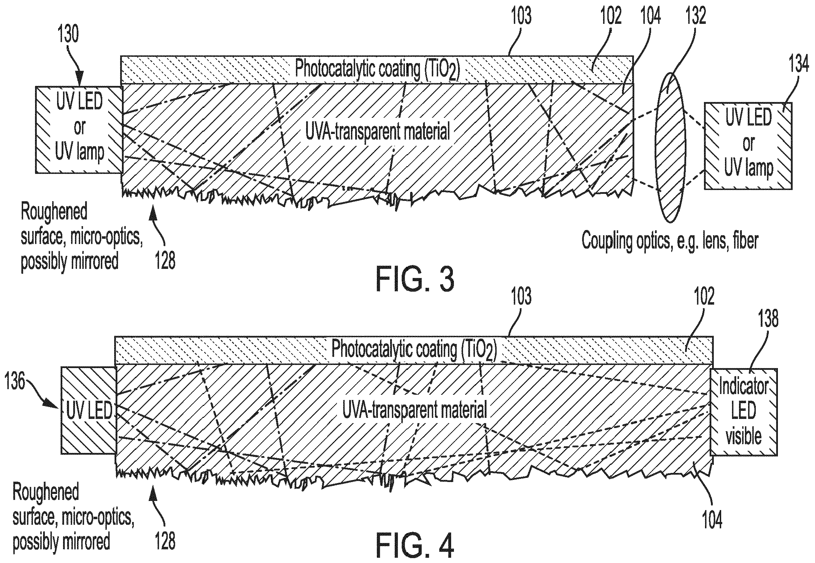

FIG. 3 illustrates a side view of a portion of the system 100 with side illumination of the surface 103, in accordance with an alternative embodiment. In the alternative embodiment shown in FIG. 3, the system 100 can be modified to include a light source 130, which may be, for example, a UV LED or a UV lamp. The light source 130 can be located at the left side of the transparent window 104, which in the embodiment shown in FIG. 3 may be configured from a UV-A-transparent material. Coupling optics 132 may be disposed to the right of the transparent window 104 and/or to the left of a side positioned light source 134 (which may be a UV LED or a UV lamp). Furthermore, the transparent window 104 may be configured with a roughened surface 128 or along with micro-optics and in some cases, a mirrored surface. Note that in the figures illustrated and discussed herein, identical or similar parts or elements are indicated by identical reference numerals.

FIG. 4 illustrates a side view of a portion of the system 100 for disinfecting a surface, wherein the system 100 can include side illumination of the surface 103 and an indicator light 138 for status display, in accordance with another embodiment. In the embodiment shown in FIG. 4, a light source 136 may be located at the left side of the transparent window 104, and the indicator light 138 may be positioned at the right side of the transparent window 104.

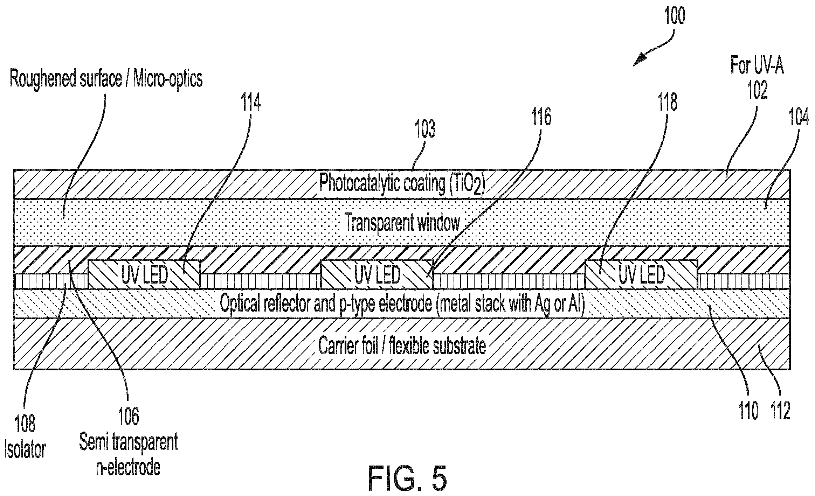

FIG. 5 illustrates a side view of the system 100 for disinfecting the surface 103, wherein the system 100 includes LED illumination of the surface 103, in accordance with an embodiment. In the embodiment shown in FIG. 4, the light source includes UV LED 114, UV LED 116, and UV LED 118 disposed partially in the isolator layer 108 and the semi-transparent n-electrode 106. Note that each UV LED 114, UV LED 116, and UV LED 118 can be implemented as a UV-A light source or UV-C light source. That is, in some embodiments, the UV light provided by UV LED 114, UV LED 116, and UV LED 118 may include UV-A light photons. In other embodiments, the UV light may provide UV-C light photons having an emission in a range of, for example, between 200nm to 280nm.

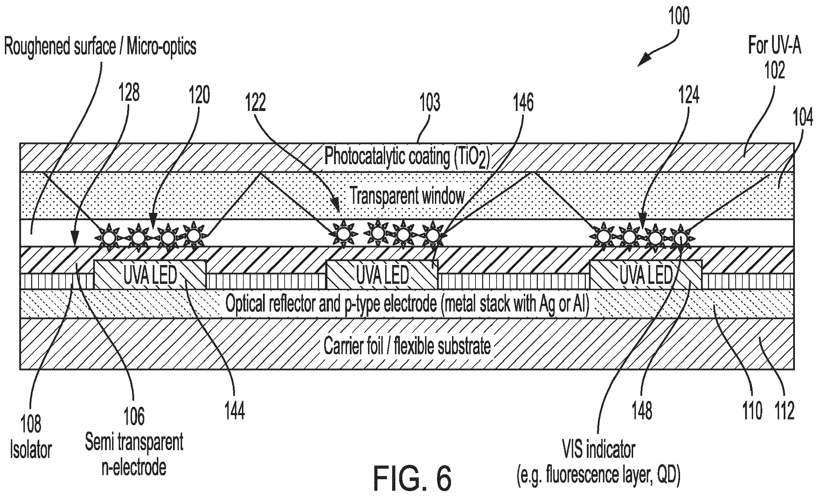

FIG. 6 illustrates a side view of the system 100 for disinfecting the surface 103, wherein the system 100 includes LED illumination with an indicator for status display, in accordance with an embodiment. In the embodiment shown in FIG. 6, the light source can include a UV-A LED 144, a UV-A LED 146, and a UV-A LED 148 disposed partially in the isolator layer 108 and the semi-transparent n-electrode 106. An indicator layer 128 can include one or more indicators such as indicator 120, indicator 122, and indicator 124, which each may comprise a visual indicator (e.g., fluorescence, QD). The indicator layer 128 may comprise a fluorescence indication layer or a luminescence indication layer that provides a visible indication of UV photons emanating through the transparent window 104.

The indicator layer 128 can be implemented to provide a 'warning' that a disinfection operation is in process. That is, it can be appreciated that what may kill or damage germs may also be harmful to humans. UV-A light is generally less harmful than UV-C. The indication layer 128 can provide a signal that can be visible to humans, so that a "disinfection operation" is indicated and people can stay away from the surface 103 while the disinfection is taking place.

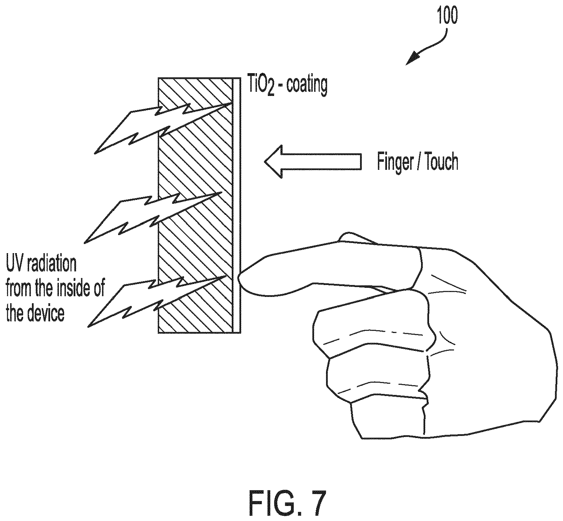

FIG. 7 illustrates a pictorial representation of the system 100, in accordance with an embodiment. As shown in FIG. 7 the system 100 can be implemented with the previously discussed TiO₂ coating to disinfect a high-touch surface of an object, such as, for example, a mobile device display, a light switch, door handle, buttons (coffee machine, elevator, etc.), doorbells, mechanical housings of any kind, touch pads, housings of medical equipment, interior of hospitals, handlebars, and so on. The system 100 can be configured in a compact implementation using, for example, III-Nitride LEDs. UV-C LED (e.g., -265nm) can lead to the destruction of RNA/DNA in germs. An alternative embodiment can involve the use of a UV-A LED light source with a color converter for white light plus the photocatalytic coating of a target surface (e.g., ZnO, TiO₂, TiO₂/Ag, etc).

Both UV-C and UV-A implementations are possible and offer various advantages and disadvantages. UV-C, for example, may require more restrictions on materials choices, and limited protection measures for UV radiation. UV-A, on the other hand, can offer a more inexpensive implementation, along with reduced safety concerns, while combining the disinfection feature with other illumination/display functionalities.

In some embodiments the disinfection cycle can be triggered after the manual operation of an electrical switch or touchpad to disinfect right after touching the surface. In other embodiments the disinfection cycle may be stopped when an internal sensor detects the proximity of a human, for example, by a motion sensor, through camera image evaluation or through any other proximity sensor that may be included in the device that may be enclosed by the self-disinfecting surface.

Based on the foregoing, it can be appreciated that a number of embodiments, preferred and alternative, are disclosed herein. For example, in a preferred embodiment, a system for disinfecting a surface can include a light source, and a transparent window located above the light source. The light source can be integrated into an object, and an outer surface of the object can be located above the transparent window. Light from the light source can irradiate the outer surface through the transparent window and from within the object to disinfect the outer surface of the object. The light can comprise violet light or ultraviolet (UV) light.

In an embodiment, a photocatalytic layer can comprise a photocatalytic material, and can be located above the transparent window and below the outer surface.

In an embodiment, the photocatalytic layer can comprise at least one of, for example, ZnO, TiO₂, Fe₂O₃, WO₃, In₂O₃, Ag, or a nonmetallic semiconductor material.

In an embodiment, the UV light can comprise at least one of, for example, UV-A light photons or UV-C light photons (i.e., having an emission in a range of between 100nm to 280nm).

In an embodiment, the light source can comprise one or more of, for example, a semiconductor-based optoelectronic device or a gas discharge lamp.

In an embodiment, the UV light can disinfect the outer surface by one or more of: inactivating germs on the outer surface of the object through reactive species created on the outer surface or damaging of nucleic acids associated with the germs, wherein the germs comprise a least one of: bacteria, virus, or a fungi; inducing antimicrobial properties in the outer surface of the object; or inducing microbicidal properties in the outer surface of the object.

In an embodiment, the light source can comprise a plurality of UV light emitting diodes, and each UV light emitting diode among the plurality of UV light emitting diodes can comprise a III-Nitride based UV light emitting diode.

An embodiment of the system can further include an isolator layer, and an electrode layer comprising a semi-transparent material, wherein the transparent window is located above the electrode layer, and the light source is disposed in the isolator layer and at least partially within the electrode layer.

In an embodiment, the photocatalytic material of the photocatalytic layer can comprise a photocatalytic coating, wherein the microbicidal properties can be induced in the outer surface when the UV light is absorbed in the photocatalytic material.

An embodiment can further include an upconversion layer that can facilitate the use of visible light to generate UV photons in the upconversion layer.

An embodiment can further include a fluorescence indication layer or a luminescence indication layer that can provide a visible indicator of UV photons emanating through the transparent window.

In an embodiment, the light source can be automatically turned off when at least one person is detected within a predetermined distance of the light source.

In an embodiment, an indication light can indicate when the outer surface of the objected has been disinfected.

An embodiment can further include a substrate upon which a plurality of layers can be located including at least the light source, the transparent window, and the outer surface.

In an embodiment, the substrate can comprise a flexible substrate.

In an embodiment, the light source can further comprise at least one of the following: a back-illumination light source with respect to the object; or a side-coupling light source with respect to the object, wherein the back-illumination light source and the side-coupled light source each comprise at least one of: a structured optical surface or an optically structured film, wherein the structured optical surface or the optically structured film promote homogenization and uniform illumination across the photocatalytic surface.

In another embodiment, a system for disinfecting a surface, can include a substrate upon which a plurality of layers are located; a transparent window located above a light source, wherein the light source is integrated into an object; an outer surface of the object located above the transparent window; and a photocatalytic layer comprising a photocatalytic material, the photocatalytic layer located above the transparent window and below the outer surface, wherein the plurality of layers includes at least the light source, the transparent window, the photocatalytic layer, and the outer surface, and wherein light from the light source excites the outer surface through the transparent window and from within the object to disinfect the outer surface of the object, the light comprising violet light or ultraviolet (UV) light.

In still another embodiment, a method for disinfecting a surface, can involve: exciting an outer surface of an object with light from a light source through a transparent window and from within the object, wherein the transparent window is located above the light source and the light source is integrated into the object, the outer surface of the object located above the transparent window, the light comprising violet light or ultraviolet (UV) light.

It will be appreciated that variations of the above-disclosed and other features and functions, or alternatives thereof, may be desirably combined into many other different systems or applications. It will also be appreciated that various presently unforeseen or unanticipated alternatives, modifications, variations or improvements therein may be subsequently made by those skilled in the art which are also intended to be encompassed by the following claims.

## Claims

1. A system for disinfecting a surface, comprising:
a light source;
a transparent window located above the light source, wherein the light source is integrated into an object; and
an outer surface of the object located above the transparent window, wherein light from the light source irradiates the outer surface through the transparent window and from within the object to disinfect the outer surface of the object, the light comprising violet light or ultraviolet (UV) light.

2. The system of claim 1 further comprising a photocatalytic layer comprising a photocatalytic material, the photocatalytic layer located above the transparent window and below the outer surface, optionally wherein the photocatalytic layer comprises at least one of: ZnO, TiO₂, Fe₂O₃, WO₃, In₂O₃, Ag, or a nonmetallic semiconductor material.

3. The system of claim 2 further comprising:
an isolator layer; and
an electrode layer comprising a semi-transparent material, wherein the transparent window is located above the electrode layer, and the light source is disposed in the isolator layer and at least partially within the electrode layer.

4. The system of claim 2 or claim 3 wherein the photocatalytic material of the photocatalytic layer comprises a photocatalytic coating, wherein the microbicidal properties are induced in the outer surface when the UV light is absorbed in the photocatalytic material.

5. The system of any one of claims 2 to 4 further comprising at least one of:
(i) an upconversion layer that facilitates a use of visible light to generate UV photons in the upconversion layer; or
(ii) a fluorescence indication layer or a luminescence indication layer that provides a visible indicator of UV photons emanating through the transparent window.

6. The system of any preceding claim wherein the UV light comprises at least one of:
UV-A light photons; or
UV-C light photons.

7. The system of any preceding claim wherein the light source comprises at least one of:
(i) a semiconductor-based optoelectronic device, or
(ii) a gas discharge lamp, or
(iii) a plurality of UV light emitting diodes, wherein each UV light emitting diode among the plurality of UV light emitting diodes comprises a group III-Nitride based UV light emitting diode.

8. The system of any preceding claim wherein the UV light disinfects the outer surface by at least one of:
inactivating germs on the outer surface of the object through reactive species created on the outer surface or damaging of nucleic acids associated with the germs, wherein the germs comprise a least one of: bacteria, virus, or a fungi;
inducing antimicrobial properties in the outer surface of the object; or
inducing microbicidal properties in the outer surface of the object.

9. The system of any preceding claim wherein the light source is automatically turned off when at least one person is detected within a predetermined distance of the light source.

10. The system of any preceding claim further comprising an indication light that indicates when the outer surface of the objected has been disinfected.

11. The system of any preceding claim further comprising a substrate upon which a plurality of layers are located including the light source, the transparent window, and the outer surface, optionally wherein the substrate comprises a flexible substrate.

12. The system of any preceding claim wherein the light source further comprises at least one of the following:
a back-illumination light source with respect to the object; or
a side-coupling light source with respect to the object, wherein the back-illumination light source and the side-couple light source each comprise at least one of: a structured optical surface or an optically structured film, wherein the structured optical surface or the optically structured film promote homogenization and uniform illumination across the photocatalytic surface.

13. A system for disinfecting a surface, comprising:
a substrate upon which a plurality of layers are located;
a transparent window located above a light source, wherein the light source is integrated into an object;
an outer surface of the object located above the transparent window; and
a photocatalytic layer comprising a photocatalytic material, the photocatalytic layer located above the transparent window and below the outer surface, wherein the plurality of layers includes at least the light source, the transparent window, the photocatalytic layer, and the outer surface, and wherein light from the light source excites the outer surface through the transparent window and from within the object to disinfect the outer surface of the object, the light comprising violet light or ultraviolet (UV) light.

14. A method for disinfecting a surface, comprising:
exciting an outer surface of an object with light from a light source through a transparent window and from within the object, wherein the transparent window is located above the light source and the light source is integrated into the object, the outer surface of the object located above the transparent window, the light comprising violet light or ultraviolet (UV) light, optionally wherein a photocatalytic layer comprising a photocatalytic material is located above the transparent window and below the outer surface, the photocatalytic layer optionally comprising at least one of: ZnO, TiO₂, Fe₂O₃, WO₃, In₂O₃, Ag, or a nonmetallic semiconductor material.

15. The method of claim 14 wherein:
the UV light comprises at least one of:
UV-A light photons;
UV-C light photons; and
the UV light source comprises at least one of: a semiconductor-based optoelectronic device or a gas discharge lamp.
